# EUROPÄISCHE PATENTSCHRIFT

(11) **EP 1 409 707 B1**
(45) Veröffentlichungstag und Bekanntmachung des Hinweises auf die Patenterteilung: **31.12.2008**
(21) Anmeldenummer: 02735162.6
(22) Anmeldetag: 22.03.2002
(51) Int. Cl.: C12P 17/12, C07D 239/06

(54) **VERFAHREN ZUR GEWINNUNG VON WERTSTOFFEN AUS ORGANISMEN**
METHOD FOR OBTAINING USEFUL PRODUCTS FROM ORGANISMS
PROCEDE POUR PRODUIRE DES SUBSTANCES UTILES A PARTIR D'ORGANISMES

(30) Priorität: 23.03.2001 DE 10114189
(43) Veröffentlichungstag der Anmeldung: 21.04.2004
(73) Patentinhaber: Bitop Aktiengesellschaft Für Biotechnische Optimierung, 58453 Witten (DE)
(72) Erfinder: KUNTE, Hans, Jörg, 53175 Bonn (DE); GALINSKI, Erwin, A., 48308 Senden (DE); GRAMMANN, Katrin, 53113 Bonn (DE); VOLKE, Angela, 53115 Bonn (DE); BESTVATER, Thorsten, 48161 Münster (DE)
(74) Vertreter: Thiel, Christian
(86) Internationale Anmeldenummer: PCT/EP2002/003252
(87) Internationale Veröffentlichungsnummer: WO 2002/077254

(56) Entgegenhaltungen:
- DE-A- 19 925 615
- GRAMMANN K ET AL: "Identification and characterization of an osmoregulated tetrahydropyrimidine transport system in Halomonas elongata." COMPARATIVE BIOCHEMISTRY AND PHYSIOLOGY PART A MOLECULAR & INTEGRATIVE, Bd. 126A, Nr. Suppl. 1, Juli 2000 (2000-07), Seite S84 XP002216027 21st Congress of the European Society for Comparative Physiology and Biochemistry;Liege, Belgium; July 24-28, 2000, July, 2000 ISSN: 1095-6433
- PETER H ET AL: "CORYNEBACTERIUM GLUTAMICUM IS EQUIPPED WITH FOUR SECONDARY CARRIERSFOR COMPATIBLE SOLUTES: IDENTIFICATION, SEQUENCING, AND CHARACTERIZATION OF THE PROLINE/ECTOINE UPTAKE SYSTEM, PROP, AND THE ECTOINE/PROLINE/GLYCINE BETAINE CARRIER, ECTP" JOURNAL OF BACTERIOLOGY, WASHINGTON, DC, US, Bd. 180, Nr. 22, 1998, Seiten 6005-6012, XP000917352 ISSN: 0021-9193
- GOUESBET GWENOLA ET AL: "Characterization of the Erwinia chrysanthemi osmoprotectant transporter gene ousA." JOURNAL OF BACTERIOLOGY, Bd. 178, Nr. 2, 1996, Seiten 447-455, XP002216028 ISSN: 0021-9193
- FORWARD JASON A ET AL: "TRAP transporters: A new family of periplasmic solute transport systems encoded by the dctPQM genes of Rhodobacter capsulatus and by homologs in diverse gram-negative bacteria." JOURNAL OF BACTERIOLOGY, Bd. 179, Nr. 17, 1997, Seiten 5482-5493, XP002216029 ISSN: 0021-9193 in der Anmeldung erwähnt
- SAUER T ET AL: "BACTERIAL MILKING: A NOVEL BIOPROCESS FOR PRODUCTION OF COMPATIBLE SOLUTES" BIOTECHNOLOGY AND BIOENGINEERING, INTERSCIENCE PUBLISHERS, LONDON, GB, Bd. 57, Nr. 3, 1998, Seiten 306-313, XP000919323 ISSN: 0006-3592
- RABUS RALF ET AL: "TRAP transporters: An ancient family of extracytoplasmic solute-receptor-dependent secondary active transporters." MICROBIOLOGY (READING), Bd. 145, Nr. 12, Dezember 1999 (1999-12), Seiten 3431-3445, XP002216030 ISSN: 1350-0872
- LOUIS P ET AL: "Characterization of genes for the biosynthesis of the compatible solute ectoine from Marinococcus halophilus and osmoregulated expression in Escherichia coli." MICROBIOLOGY (READING, ENGLAND) ENGLAND APR 1997, Bd. 143 ( Pt 4), April 1997 (1997-04), Seiten 1141-1149, XP002216031 ISSN: 1350-0872 in der Anmeldung erwähnt
- GRAMMANN KATRIN ET AL: "New type of osmoregulated solute transporter identified in halophilic members of the Bacteria domain: TRAP transporter TeaABC mediates uptake of ectoine and hydroxyectoine in Halomonas elongata DSM 2581T." JOURNAL OF BACTERIOLOGY, Bd. 184, Nr. 11, Juni 2002 (2002-06), Seiten 3078-3085, XP008009093 June, 2002 ISSN: 0021-9193

## Beschreibung

Die Erfindung betrifft ein Verfahren zur permanenten Produktion von Wertstoffen in Organismen, die über genetische Informationen zur Produktion der Wertstoffe und Regelmechanismen zur Steuerung der Produktion verfügen. Unter "Werstoffen" werden erfindungsgemäß Ectoin und /oder Hydroxyectoin verstanden.

Regelmechanismen zur Limitierung der Biosynthese sind einerseits Aufnahmetransport-Systeme und andererseits Stoffwechselengpässe. Die Aufnahmetransport-Systeme führen den bereits erzeugten Wertstoff wieder in die Zelle zurück und geben somit das Signal für den Biosynthese-Stop. Um diesen Mechanismus zu umgehen, werden entweder diese "natürlichen" Aufnahme-transport-Systeme ausgeschaltet oder das für die Synthese erforderliche Gen in einen Fremdorganismus ohne Aufnahmetransport-Systeme oder zumindest mit einem defekten Transport-System eingebracht. In Fremdorganismen sind jedoch unter Umständen Stoffwechselengpässe vorhanden, die auch die Biosynthese limitieren.

Unter dem Begriff "permanente Produktion" wird das kontinuierliche Abfließen der Wertstoffe aus den Zellen verstanden, wobei die Zellen unabhängig von deren Wachstum und Vermehrung neue Wertstoffe aufgrund des Verlustes wieder produzieren. Die permanente Produktion kann sowohl in wachsenden bzw. sich vermehrenden als auch in nicht wachenden bzw. sich nicht vermehrenden Zellen durchgeführt werden.

Die biotechnologische Produktion von Wertstoffen zielt darauf ab, diese Wertstoffe durch Eigensynthese (bzw. Biokonversion aus Vorstufen) oder durch rekombinante Expression von Stoffwechselwegen in Produktionsstämmen in hoher Konzentration in den Zellen oder im Medium zu erhalten. Sofern die Produkte in den Zellen angereichert sind, besteht der Stand der Technik für die Gewinnung darin, die Zellen entweder aufzuschließen, zu permeabilisieren oder auf andere Weise zur Abgabe der Wertstoffe zu veranlassen (s. "Bakterienmelken"). Nachteilig ist die geringe Endkonzentration der Wertstoffe in der Produktlösung und die damit verbundenen hohen Kosten für die Produktaufreinigung ("downstream processing").

DE 199 25 615 A beschreibt ein Verfahren zur Produktion von Ectoin in nicht-halophilen Organismen. Um diese Organismen zur Ectoinproduktion zu befähigen, werden Ectoingene aus halophilen Organismen extrahiert und in die produzierenden Mikroorganismen übertragen. In den produzierenden Mikroorganismen fehlen die Stoffwechselengpässe entweder ganz oder aber sind ausgeschaltet.

K. Grammann, A. Volke und H. J. Kunte, Journal of Bacteriology, Bd. 184, Nr. 11, Juni 2002, Seiten 3078-3085 beschreiben erstmalig einen Tetrahydropyrimidintransporter aus einem halophilen Bakterium und ein osmoreguliertes Aufnahmesystem, das mit der TRAP-Transporterfamilie zusammenhängt.

R. Rabus et al., Microbiology (Reading), Bd. 145, Nr. 12, Dezember 1999, Seiten 3431-3445 beschreiben den Transport von C4-Dicarboxylaten durch TRAP-Transporter und Deletionen in den Transportern.

Gegenstand der vorliegenden Erfindung ist nun ein Verfahren nach Anspruch 1, das die Gewinnung von Wertstoffen dadurch ermöglicht, daß hochaffine Aufnahmesysteme für diese Wertstoffe ausgeschaltet oder in ihrer Funktion beeinträchtigt werden bzw. Produktionsstämme verwendet werden, denen besagte Aufnahmesysteme fehlen. Die zugrundeliegende überraschende Entdeckung ist, daß das beobachtete Konzentrationsgefälle über der Zellmembran nicht allein dadurch aufrecht erhalten wird, daß die Membranen impermeabel für den Wertstoff sind, sondern auch dadurch, daß hochaffine Aufnahmesysteme "ausgelaufene" Wertstoffe rasch wieder in die Zelle hineintransportieren. Auf diese Weise entsteht eine nur scheinbar stationäre Situation, denn in Wirklichkeit handelt es sich um ein ständiges Rezyklisieren von "abgeflossenen" Wertstoffen. Die erfinderische Idee besteht darin, diese Wiederaufnahmesysteme auszuschalten oder zu beeinträchtigen, so daß die produzierten Wertstoffe dem Konzentrationsgradienten folgend kontinuierlich in das umgebende Medium abfließen. Aufgrund der eigenen Regulationsmechanismen werden die abgeflossenen Substanzen ergänzt. Diese Regenerierung verlorener Wertstoffe ist unabhängig von Wachstum und Vermehrung der Zellen und kann auch mit nicht wachsenden bzw. sich nicht vermehrenden Zellen durchgeführt werden. Unter geeigneten Bedingungen handelt es sich um eine Konversion von Substrat zu dem gewünschten Wertstoff und entspricht dem Prozeß einer Biokatalyse. Je nachdem, ob die Neusynthese mit der Geschwindigkeit des Abflusses Schritt halten kann oder nicht, wird sich in den Zellen eine Konzentration des Wertstoffes einstellen, die der natürlichen Konzentration entspricht oder darunter liegt. Im Gleichgewichtszustand entspricht die Biosyntheseleistung der Produktgewinnungsrate. Das erfindungsgemäße Verfahren läßt sich z. B. auch auf die Produktion von osmotisch induzierten Wertstoffen unter niedrig-osmotischen Bedingungen (salzarme Medien) anwenden. Die maximal im Medium erreichbare Konzentration entspricht der (unter den gegebenen Bedingungen) maximal in der Zelle erreichbaren Konzentration. Sie kann im günstigsten Fall mit herkömmlichen Fermentationstechniken molare Dimensionen erreichen.

Mit Hinblick auf eine möglichst hohe Raum-Zeit-Ausbeute wird das erfindungsgemäße Verfahren durch geeignete Maßnahmen ergänzt, die die Eigenbiosynthese der Wertstoffe unterstützen bzw. (im Falle der Übertragung eines Stoffwechselweges auf einen entsprechenden Produzentenstamm) mögliche Stoffwechselengpässe beseitigen. Dies ist z. B. dadurch zu realisieren, daß die Medien- und Wachstumsbedingungen variiert werden bzw. die übertragenen Stoffwechselwege mit weiteren Genen (Helfer-Genen) kombiniert werden, die regulatorische oder funktionale Engpässe beseitigen.

Produkte, die nach erfindungsgemäßem Verfahren gewonnen werden können, sind die kompatiblen Solute aus der Gruppe der Ectoine zu nennen, die entweder als reine Substanz oder in Kombination mit anderen Stoffen Biomoleküle (Nukleinsäuren, Proteine, Lipide), komplexe "self assembly" Strukturen (z. B. Ribosomen; Protein-DNA-, Protein-Protein- und Protein-Zucker-Komplexe; Lipidfilme, Membranen etc.) oder auch ganze Zellen stabilisieren [da Costa et al. 1997]. Weitere Anwendungsmöglichkeiten liegen in der Verwendung als wasserbindende Substanz (moisturizer) und Wirkstoff in Haut- und Körperpflegemitteln, als Probiotika, Nutraceuticals und in pharmazeutischen Formulierungen.

Die erfindungsgemäße Ausschaltung bzw. Beeinflussung von Transportsystemen zum Zwecke der Produktion von Wertstoffen kann auf verschiedene-Weise erfolgen:
a) durch Mutation der kodierenden Gene in den produzierenden Organismen
b) durch Hemmung/Inaktivierung des Transportsytems auf Proteinebene
c) durch Gewinnung der Gene für einen bestimmten Biosyntheseweg und Übertragung auf einen Produktionsstamm, der entweder natürlicherweise keine entsprechenden Transportsysteme hat oder bei dem diese ausgeschaltet wurden

Die Gene, die für die spezifischen Wertstofftransporter kodieren, können mit Hilfe verschiedener genetischer Methoden mutiert werden, z. B. Transposonmutagenese oder gezielte Mutation (z. B. Deletionen) durch PCR-Techniken. Es sollte als Produktionsstamm für die Gewinnung von Wertstoffen ein Organismus gewählt werden, der möglichst wenige Tranporter für die zu produzierende Substanz besitzt. Ein solcher genetischer Hintergrund hat den Vorteil, daß u. U. nur ein Transporter ausgeschaltet werden muß und somit eine Produktionsstamm konstruiert wird, der unter keiner der gewählten Anzuchtbedingungen den Wertstoff wieder aufnehmen und metabolisieren kann. Dies erweitert die Anzuchtmöglichkeiten und Produktionstechniken des erfinderischen Verfahrens.

Alternativ ist eine Beeinträchtigung der Transportsysteme dadurch möglich, daß Antimetabolite dem Medium zugesetzt werden, die entweder an den Transporter binden (ohne transportiert zu werden) und dadurch oder auf andere Weise eine Veränderung des Transportporteins bewirken, so daß es nicht mehr oder nur noch in eingeschränktem Maße in der Lage ist, die Funktion des Wertstofftransports zu übernehmen.

Die Übertragung von Genen für die Wertstoffproduktion wird betrieben, um bekannte Produzentenstämme wie z. B. *E. coli, Bacillus subtilis* und *Corynebacterium glutamicum* einsetzen zu können, und auf diese Weise unabhängig von den natürlichen Produzentenstämmen zu werden, die sich .z. T. sehr schlecht kultivieren lassen oder ungewöhnliche Anzuchtbedingungen erfordern.

Im Falle der Ectoinproduktion wird eine Übertragung der für die Syntheseenzyme des Ectoin kodierenden Gene auf nicht-halophile Produzentenstämme (wie z. B. *E.coli*) angestrebt, um die in der Verwendung salzhaltiger Medien begründeten Korrosions- und Entsorgungsprobleme zu lösen. Bei der gentechnischen Übertragung eines ganzen Stoffwechselweges ist jedoch die komplexe Regulation in dem Empfängerorganismus zu beachten. So können die Ectoingene zwar auch in nicht-halophilen Organismen exprimiert werden [Galinski & Louis 1997, Louis & Galinski 1997], die Produktionsrate ist aber auch dort osmotisch reguliert und durch metabolische Engpässe (z. B. auf der Stufe der Aspartatkinase) limitiert.

Für die Durchführung des erfindungsgemäßen Verfahrens ist die Kultivierung in einem Fermenter vorteilhaft. Dabei sind die Randbedingungen so zu wählen, daß die Kultur möglichst lange in der Wachstumsphase verweilt und/oder hohe Zelldichten erreicht werden (z. B. Hochzelldichte-Fermentation) oder die Zellen durch Nährstofflimitierung am Wachstum gehindert werden, so daß die angebotene C-Quelle möglichst vollständig in Wertstoff umgesetzt wird. Im Falle der Übertragung eines Biosynthesewegs für den Wertstoff auf einen geeigneten Produzentenstamm müssen zusätzlich geeignete Bedingungen geschaffen werden, um die Gene (ggf. in Kombination mit Helfergenen) zu induzieren. Die beschriebene Produktionsstrategie ließe sich auch dergestalt modifizieren, daß Zellen durch Immobilisierung oder durch eine Membran (z. B. Dialyseschlauch) von der Produktionslösung getrennt sind. Nach Erreichen der maximalen Produktkonzentration könnte die Zellmasse in neues Medium übertragen werden ("Teebeutel-Prinzip") und so der Prozeß kontinuierlich fortgesetzt werden. Das geschilderte Verfahren bietet sich insbesondere dann an, wenn der Wertstoff über einen energieunabhängigen Syntheseweg gebildet wird (z. B. das Aminosäurederivat Ectoin). In diesem Fall ist es möglich, die angebotene C-Quelle *vollständig* in Wertstoff umzuwandeln ("Biokonversion"), so daß der Organismus die Funktion eines Biokatalysators übernimmt.

Ist ein optimaler Produktionsstamm gewählt bzw. konstruiert worden, ist es mit den beschriebenen Produktionstechniken möglich, den Wertstoff in hoher Konzentration (bis zu 0.5 M und mehr) außerhalb der Zelle anzureichern, ohne daß die Zellen aus dem Medium abgeerntet und aufgeschlossen, vorübergehend permeabilisiert oder auf andere Weise (z. B. Verdünnungsstreß) zur Abgabe der Substanzen veranlaßt werden müßten. Idealerweise verbleiben die Produktionszellen solange im Medium bis die Medienbestandteile (z. B. C-Quelle) vollständig verbraucht sind, um die anschließende Aufreinigung zu vereinfachen.

Für den Fall, daß der Wertstoff nur unzureichend über die Membran abfließen sollte, ließe sich das Verfahren für z. B. osmophile oder halophile Organismen auch so modifizieren, daß die Zellen einem Verdünnungsstreß ausgesetzt werden. Dieser osmotische "downshock" induziert die Abgabe der Wertstoffe in das Medium. Gegenüber dem herkömmlichen "Bakterienmelken" besitzt das hier beschriebene Verfahren den Vorteil, daß Produktionszellen und Produkt nach der Produktabgabe nicht mehr getrennt werden müssen, da die Zellen die Fähigkeit verloren haben, die Wertstoffe wieder aufzunehmen. Vielmehr kann direkt im Anschluß an den osmotischen Verdünnungsstreß die Konzentration an gelösten Stoffen (z. B. NaCl, PEG etc.) wieder erhöht werden ("upshock") und die erneute Produktion von Wertstoffen eingeleitet werden. Ähnlich wie das klassische "Bakterienmelken" kann auch dieser Prozeß kontinuierlich gestaltet werden.

Das physiologische Prinzip, das der Erfindung zugrundeliegt und es ermöglicht, durch Veränderung bzw. Beeinträchtigung von Solutetransportern Wertstoffe zu gewinnen, soll anhand des erfindungsgemäßen TRAP-Transporters (TRAP-T) mit dem Namen TeaABC aus Halomonas elongata erklärt werden.

TRAP-T-Systeme sind Transporter, die ihre Energie für den Transport von Stoffen aus dem Membranpotential der Zelle beziehen und daher als sekundäre Transporter bezeichnet werden. TRAP-T Systeme verfügen über ein Substratbindeprotein, das außerhalb der Zelle lokalisiert ist und eine Membrandomäne. Die Membrandomäne der TRAP-T Syteme besteht entweder aus einem kleinen und einem großen Transmembranprotein oder einem Fusionsprotein der beiden Membranproteine. Das Transporter TeaABC aus H. elongata ist ein TRAP-T System mit zwei Membranprotein. TeaABC transportiert die kompatiblen Solute Ectoin und Hydroxyectoin (Wertstoffe) bei osmotischem Streß in das Zellinnere (Cytoplasma) und ermöglicht die Anreicherung dieser Substanzen in hoher molarer Konzentration, um ein osmotisches Gleichgewicht zwischen der Zelle und dem Außenmedium herzustellen. TeaABC wird durch osmotischen Streß aktiviert, es handelt sich um ein osmoreguliertes Transportsystem. Die Hauptaufgabe von TeaABC besteht nun aber nicht darin zellfremdes Ectoin aus dem Medium aufzunehmen. Dies wird zum einen dadurch erklärt, daß Ectoin im Medium natürlicherweise nicht in hoher Konzentration vorkommt. Außerdem zeigte sich, daß die Zerstörung des Transporters zu einem kontinuierlichen Verlust von Ectoin führt. Offensichtlich wird TeaABC hauptsächlich dafür verwendet, zelleigenes Ectoin wieder in die Zelle zurück zu transportieren. Der Verlust von Ectoin ist nun aber kein unspezifischer Vorgang. Vielmehr scheint es so, daß das Solut über eine spezielle Pore die Zelle verläßt, und zwar dann, wenn ausreichend Ectoin im Zellinneren durch Eigensysnthese vorhanden ist. Außerhalb der Zelle (Periplasma) angelangt, wird Ectoin durch das Substratbindeprotein TeaA des Transporters TeaABC gebunden und wieder in die Zelle transportiert. Der Transport von Ectoin zurück in die Zelle dient als Signal, daß ausreichend kompatibles Solut Ectoin synthetisiert wurde und die Ectoinsysnthese der Zelle wird daraufhin eingestellt. Als Vermittler zwischen dem aktiven Transporter und der Synthese von Ectoin dient ein Regulatorprotein mit dem Namen TeaD. TeaD wird von einem Gen kodiert, das neben den Genen des Transporters TeaABC liegt und wahrscheinlich zusammen mit diesen exprimiert wird. TeaD muß mit dem Transporter TeaABC interagieren und erkennen, daß das Transportsystem Ectoin aufnimmt. Diese Aktivität wird wahrscheinlich durch Phosphorylierung weitergeleitet und führt zur entsprechenden negativen Regulation der Ectoin-Synthese. Die Bedeutung von TeaD als Regulator wurde durch Mutationsexperimente bestätigt. Die Zerstörung von TeaD führt bei aktiven Transporter TeaABC dazu, daß Ectoin aus der Zelle fließt. Obwohl TeaABC Ectoin transportiert, kann dieses Signal nicht weitergeleitet werden und die Synthese von Ectoin verweilt auf zu hohem Niveau. Eine Zerstörung bzw. Beeinträchtigung von TeaABC führt somit nicht nur dazu, daß Ectoin die Zelle verläßt, sondern auch dazu, daß der Verlust durch vermehrte Synthese ausgeglichen wird da die Zelle kein negatives Signal durch TeaABC und TeaD erhält. Mutanten, wie sie in Verfahrensbeispiel 1 dargestellt werden, sind daher sowohl Ausscheidermutanten als auch Überproduzenten für Wertstoffe.

Das Ziel, Wertstoffe mittels veränderter bzw. beeinträchtigter Transportsysteme zu gewinnen kann auch dadurch erreicht werden, dass die Biosynthesegene für die gewünschten Wertstoffe in Produzentenstämmen mit entsprechend veränderten/beeinträchtigten oder ganz fehlenden Transportsystemen exprimiert werden. Der Vorteil besteht darin, dass nur einmal ein geeigneter Produzentenstamm erzeugt werden muss (z.B. ein E.coli-Stamm, dem sämtliche osmoregulierten Transportsysteme fehlen), in dem dann verschiedene Wertstoffe produziert werden können.

Während die rekombinante Expression von einzelnen Fremdgenen in Pröduzentenstämmen grundsätzlich beherrscht wird, entsteht bei der Übertragung von ganzen Stoffwechselwegen allerdings immer das Problem, dass auch Regulationsaspekte der enzymatischen Kaskade (z.B. die Regulationen der cytoplasmatischen Konzentration von Vorstufen) mit einbezogen werden müssen. Für den Fall der Übertragung des Genclusters für die Ectoin-biosynthese bedeutet dies, dass der biosynthetische Engpass (eine streng regulierte Aspartokinase) für diesen Biosyntheseweg ("Flaschenhals") erst durch die Einbringung eines weiteren Gens, nämlich eine deregulierte Aspartokinase, geöffnet werden kann.

### Verfahrensbeispiel 1

### Gewinnung des Wertstoffes Ectoin aus dem Eubakterium Halomonas elongata DSM 2581^{T} durch Mutation des Ectointransporters TeaABC

### (A) Konstruktion einer Ectoin-Transportmutante von Halomonas elongata

Das Gen *tea*C, das für die große Transmembranuntereinheit des osmoregulierten Ectoin-Transporters TeaABC aus TRAP-T-Familie kodiert [Forward et al. 1997], wird mit Hilfe der SOE-PCR-Technik (splicing by overlap extenstion) deletiert. Das resultierende DNA-Fragment mit den nun verbundenen Sequenzen stromaufwärts und -abwärts von *teaC* wird mit Hilfe des konjugativen Plasmids pK18*mobsacB* [Schäfer et al.] in den Wildtyp von *H*. *elongata* eingebracht. Durch homologe Rekombination erfolgt ein Austausch der Wildtyp-DNA gegen die des Deletionsfragmentes. Die daraus hervorgehende Deletionsmutante *H. elongata* KB2 (Δ*tea*C) ist nicht mehr in der Läge, Ectoine bei osmotischen Streß aufzunehmen.

### (B) Kulturmedium

Glucose-Mineralsalzmedium (VVM):NaCl (100 g/l); KCI (1 g/l); MgSO₄x7H₂O (6,5 g/l); NH₄Cl (3 g/l); K₂HPO₄ (0,5 g/l); CaCl₂x2H₂O (0,01); FeSO₄x7H₂O (0,01); D-GlucosexH₂O (11 g/l), Hefeextrakt (0,1 g/l), Spurenelementlösung (TES; Claus et al. 1983) (1 ml/l); Vitaminlösung-VA (Imhoff & Trüper 1977) (1 ml/l)

### (C) Kulturbedingungen

*H. elongata* KB2 (Δ*tea*C) wird in einem Biostat V-Fermenter mit einem Arbeitsvolumen von 4 I bei 30°C in einem fed-batch Prozeß angezogen. Die GlucoseKonzentration in der Kulturlösung wird durch Zugabe einer konzentrierten fed-batch Lösung (594,6 g/l Glucose-monohydrat; 133,6 g/l NH₄Cl; 87 g/L K₂HPO₄; 100 g/L NaCl) zwischen 5 g/L und 15 g/L gehalten. Die Kultur wird mit Druckluft belüftet (3 l/min), die Rührerdrehzahl beträgt 250 U/min. Die Regulation des pH-Werts auf pH 7,2 erfolgt durch die sterile Zugabe von HCl (1 M) oder NaOH (5 M) über Schlauchpumpen. Die Zunahme der Zellzahl wird durch Bestimmung des Zellproteingehalts und die Messung der optischen Dichte bei 600 nm (OD₆₀₀) verfolgt.

### (D) Probennahme, Analytik

Die Proben zur Bestimmung des Glucosegehalts im Medium, des Zellproteingehalts, der OD₆₀₀ und des Ectoingehalts im Medium werden über eine Kanüle im Fermenterdeckel abgenommen. Zur Bestimmung des Ectoingehalts im Medium werden die Zellen abzentrifugiert und die Medienüberstände auf einer isokratischen HPLC mit UV-Detektion vermessen.

### (F) Anreicherung von Ectoin im Medium

Ectoin (aber kein Hydroxyectoin) reichert sich im Verlauf des gesamtem Fermentationsprozesses im Medium an. Mit Erreichen der maximalen OD₆₀₀ von 54,0 verliert der Organismus ca. 8,2 g Ectoin.. Damit befindet sich ca. 60% des gesamten Ectoins im Medium. Auch in der stationären Phase synthetisiert *H. elongata* KB2 Ectoin, und die finale Konzentration an externem Ectoin beträgt 15 g/l (ca. 100 mM). Damit sind ca. 90% des gebildeten Ectoins in das Medium entlassen worden. Mit Verbrauch der C-Quelle Glucose stellt *H*. *elongata* die Produktion von Ectoin ein.

### Verfahrensbeispiel 2

### Gewinnung des Wertstoffes Ectoin durch Konstruktion eines Vektors, der die Ectoinsynthesegene mit einem Helfer-Gen (deregulierte Asparatkinase) kombiniert zur Übertragung auf transportdefekte Produktionsstämme

Durch die Offenlegungsschrift DE 199 25 615 A1 (7.12.2000) wird ein Verfahren offenbart, das geeignet ist, den kompletten Ectoinbiosyntheseweg unter gleichzeitiger Öffnung von möglichen Stoffwechselengpässen auf andere Organismen (z. B. *E. coli* DH5α) zu übertragen und auf diese Weise Ectoin in salzarmen Medien zu produzieren. Entgegen den Erwartungen wurde dadurch aber keine Überproduktion erzielt, und zwar vermutlich deshalb, weil den Ectoingenen ein geeigneter Promotor fehlte und weil auf Proteinebene eine Feed-Back-Regulation der Ectoin-Gene besteht. Diese Verfahrensbeispiel beschreibt die Konstruktion eines Vektors pAKECT1, in dem die Ectoin-Gene unter der Kontrolle des eigenen Promotors (ectUP) stehen und mit dem Helfer-Gen *lys*C (aus *Corynebacterium glutamicum* MH20-22B) kombiniert sind. Auf diese Weise kann unter Öffnung von-Stoffwechselengpässen in *E. co*/*i eine* Ectoin-Biosynthese erzielt werden, die in transportdefekten Produzentenstämmen zu der gewünschten Überproduktion in einem Milieu geringer Salinität führt.

### (A) Konstruktion des Vektors pAKECT1

Ein Nukleinsäure-Fragment aus dem Gram-positiven, halophilen Bakterium *Marino-coccus halophilus,* welches die Gene zur Synthese des kompatiblen Solutes Ectoin trägt (*ectA, ectB, ectC*), und ein Nukleinsäure-Fragment aus dem Gram-positiven Bakterium *Corynebacterium glutamicum* MH20-22B [Cremer et al. 1991], welches das Gen für ein gegen "feed-back" Hemmung resistentes Enzym Aspartokinase trägt (*lysC*), wurden mittels molekularbiologischer Standardverfahren aus den Plasmiden pOSM12 bzw. pRK1 in den Vektor pHSG575 ligiert. Im Gegensatz zu Plasmid pAKECT2, bei dem das Ectoin-Gencluster unter Kontrolle des *lac*-Promotors und das Gen für die Aspartatkinase unter Kontrolle des tac-Promotors steht, werden die Ectoin-Gene in Plasmid pAKECT1 durch die eigenen Regulatorsequenzen ectUP kontrolliert.

### (B) Übertragung des Vektors auf E. coli DH5α

Durch CaCl₂-Transformation [Hanahan 1983] wurde das Konstrukt in *Escherichia coli* DH5α übertragen. Die Identifikation der Klone mit Fremd-DNA erfolgte auf genetischem Wege mittels Plasmidisolierung, Restriktionsverdau und Agarose-Gelelektrophorese nach bekannten molekular-biologischen Methoden [Ausubel et al. 1992].

### (C) Bestimmung des intrazellulären Ectoingehalts in E. coli DH5α

Der das rekombinante Plasmid enthaltende Empfängerorganismus *Escherichia coli* DH5α wurde in Glucose-Mineralsalzmedium MM63 nach Larsen et al. [1987] mit verschiedenen Salzkonzentrationen (1-5 % - NaCl) kultiviert. Die Anzucht erfolgte in 250-ml Erlenmeyerkolben mit Schikane in 100 ml Medium (Rotationsschüttler Innova 4232 der Fa. New Brunswick, Edison; 150 rpm, 37 °C). Das gewonnene Zellmaterial wurde mittels Zentrifugation geemtet (4°C, 2.800 g, 15 min, Zentrifuge Universal R16 , Hettich, Tuttlingen), bei 70°C getrocknet und zur Gewinnung der intrazellulär vorliegenden kompatiblen Solute extrahiert [Galinski 1986]. Die Analyse des intrazellulären Solutespektrums erfolgte mittels isokratischer HPLC [Galinski 1986]. Dabei wurde die Ectoinproduktion von AKECT2- und AKECT1-Konstrukten vergleichen, und zwar jeweils ohne und mit IPTG-Induktion (1 mM).

Der intrazellulären Ectoingehalt ist abhängig von der Salinität des Mediums Durch die IPTG-Induktion des Helfer-Gens *lys*C in pAKECT1 wird der Stoffwechselengpaß behoben und werden deutlich höhere Ectoinmengen erzielt. Überraschenderweise sind die mit pAKECT1 erzielten Ectoinkonzentrationen nahezu identisch mit denen, die von dem Kontrollstamm pHSG575 erzielt werden, wenn Ectoin im Medium angeboten wird. Daran wird deutlich, daß das Transportsystem für Ectoin (und nicht die Biosyntheseleistung der Zellen) die interne Konzentration determiniert. Da die maximale Konzentration bei 2% NaCl schon beinahe erreicht wird, wird das Ziel einer rekombinanten Ectoin-Zellfabrik für salzarme Medien dadurch verwirklicht, daß der Vektor pAKECT1 entsprechend erfindungsgemäßem Verfahren auf Empfänger-Organismen übertragen wird, denen die Transportsysteme für Ectoin fehlen oder deren Transportsysteme ausgeschaltet oder in ihrer Funktion beeinträchtigt sind.

### LITERATUR

- Ausubel FM, Brent R, Kingston RE, Moore DD, Seidman JG, Smith JA. Struhl K (eds.) (1992) Short protocols in Molecular biology, 2nd Edition. John Wiley & Sons, New York.
- Claus D., Fahmy F., Rolf H. J., Tosunoglu N. (1983) Sporosarcina halophila sp. nov., an obligate, slightly halophilic bacterium from salt marsh soils. Syst Appl Microbiol 4:496-506;
- Cremer J, Eggeling L. Sahm H (1991) Control of the lysine biosynthesis sequence in Corynebacterium glutamicum as analyzed by overexpression of the individual corresponding genes. Appl Environ Microbiol 57: 1746-1752
- da Costa MS, Santos H, Galinski EA (1998) An overview of the role and diversity of compatible solutes in Bacteria and Archaea. Adv Biochem Eng Biotechnol 61: 117-153
- Forward, J. A., Behrendt M. C., Wybom N. R., Cross R., and Kelly D.J. (1997) TRAP Transporter: a New Family of Periplasmic Solute Transport Systems Encoded ny the dctPQM Genes of Rhodobacter capsulatus and by Homolgs in Diverse Gram-Negative Bacteria. Journal of Bacteriology 179:5482-5493;
- Galinski EA (1986) Salzadaptation durch kompatible Solute bei halophilen phototrophen Bakterien. Dissertation, Universität Bonn.
- Galinski EA, Louis P (1997) Verfahren zur Übertragung der Fähigkeit zur Bio-synthese kompatibler Solute und der damit verbundenen Streßtoleranz auf Organismen. OS DE 196 10 972 A1
- Hanahan D (1983) Studies on Transformation of Escherichia coli with Plasmids. Mol Biol 166: 557-580.
- Imhoff J. F., Trüper H. G. (1977) Ectothiorhodospira halochloris sp. nov., a new extremely halophilic phototrophic bacterium containing bacteriochlorophyll b: Arch Microbiol 114:115-121.
- Larsen PI, Sydnes LK, Landfald B, Strom AR (1987) Osmoregulation in Escherichia coli by accumulation of organic osmolytes: betaines, glutamic acid and trehalose. Arch Microbiol 147: 1-7
- Louis P, Galinski EA (1997) Characterization of genes for the biosynthesis of the compatible solute ectoine from Marinococcus halophilus and osmoregulated expression in Escherichia coli. Microbiology 143: 1141-1149
- Schäfer, A., A. Tauch, W. Jäger, J. Kalinowski, G. Thierbach, and A. Pühler. (1994) Small mobilizable multi-purpose cloning vectors derived from the Escherichia coli plasmids pK18 and pK19: selection of defined deletions in the chromosome of Corynebacterium glutamicum. Gene 145:69-73

## Patentansprüche

1. Verfahren zur permanenten Produktion von Ectoin und/oder Hydroxyectoin in Organismen, die über genetische Informationen zur Produktion von Ectoin und/oder Hydroxyectoin und Regelmechanismen zur Steuerung der Produktion verfügen, **dadurch gekennzeichnet, dass** ein vorhandener Regelmechanismus zur Limitierung der Biosynthese in den Zellen deletiert, ausgeschaltet und/oder in ihrer Funktion beeinflusst werden, wobei es sich bei dem Regelmechanismus um den TRAP-Transporter TeaABC handelt.

2. Verfahren nach Anspruch 1, **dadurch gekennzeichnet, dass** es sich bei den Produktionsstämmen um pflanzliche und/oder tierische Zellkulturen und um Hefen, Pilze oder eukaryontische Mikroorganismen handelt.

3. Verfahren nach Anspruch 1, **dadurch gekennzeichnet, dass** es sich bei den Produktionsstämmen um Prokaryonten, insbesondere um Archae und Bacteria, handelt.

4. Verfahren nach Anspruch 1, **dadurch gekennzeichnet; dass** es sich bei den Produktionsstämmen um Enterobakterien, insbesondere um E. coli, um Bacillus-Arten, um Corynebakterien und um Mikroorganismen aus der Familie. Halomonadaceae, vorzugsweise aus der Gattung Halomonas, um Marinococcus und verwandte Bakterien und um Rhodothermus handelt.

5. Verfahren nach Anspruch 1, **dadurch** gekennzeichhet, dass die Synthese mittels Erhöhung oder Erniedrigung der Temperatur oder mittels Erniedrigung der Wasseraktivität induziert wird.

6. Verfahren nach Anspruch 1, **dadurch gekennzeichnet, dass** die Synthese ohne Induktion bei konstant hoher oder bei niedriger Temperatur durchgeführt wird oder bei konstant niedriger Wasseraktivität erfolgt.

7. Verfahren nach Anspruch 1, **dadurch gekennzeichnet, dass** die Synthese durch wachsende, metabolisch aktive Zellen erfolgt.

8. Verfahren nach Anspruch 1, **dadurch gekennzeichnet, dass** die Synthese in Form einer Biokonversion mit ruhenden bzw. immobilisierten Zellen erfolgt.

9. Verfahren nach Anspruch 1, **dadurch gekennzeichnet, dass** die Wertstoffe mittels permeabler Membrane, bevorzugt mittels Dialyse-Fermenters, von den Produktionszellen getrennt sind.

10. Verfahren nach Anspruch 1, **dadurch gekennzeichnet, dass** die Beeinflussung bzw. Beeinträchtigung des Aufnahmetransport-Systems durch Mutationen mittels Deletion, Punktmutation, Insertionen, Beeinflussung und/oder Inversionen in den kodierenden Genen erfolgt.

11. Verfahren nach Anspruch 1, **dadurch gekennzeichnet, dass** die Beeinflussung bzw. Beeinträchtigung des Aufnahmetransport-Systems durch Hemmung und/oder Blockierung des Transports auf Proteinebene mittels Einsatz von Antimetaboliten oder Chemikalien erfolgt.

12. Verfahren nach Anspruch 1, **dadurch gekennzeichnet, dass** die Beeinflussung bzw. Beeinträchtigung des Aufnahmetransport-Systems durch eine Manipulation der Energieversorgung der produzierenden Zellen erfolgt.

13. Verfahren nach Anspruch 1, **dadurch gekennzeichnet, dass** es sich um TeaABC aus der Familie der Halomonadaceae, vorzugsweise aus Halomonas-Arten, handelt.

14. Verfahren nach Anspruch 1, **dadurch gekennzeichnet, dass** dem TeaABC ein Regulator TeaD zugeordnet ist, wobei dieser nach Bedarf ausgeschaltet wird.

15. Verfahren nach Anspruch 1, **dadurch gekennzeichnet, dass** durch Anzuchts- und Produktionsbedingungen der Organismen die Aufnahmetransport-Systeme nicht oder nur teilweise aktiv sind.

16. Verfahren nach Anspruch 1, **dadurch gekennzeichnet, dass** die Übertragung der Nukleinsäuren, insbesondere Gene bzw. Regulationssequenzen/Promotoren mittels Vektoren erfolgt, daß sie in dem Empfängerorganismus autonom repliziert oder dauerhaft in das Genom inseriert wird, wobei es sich bei dem Empfängerorganismus um Mikroorganismen handelt, denen osmoregulierte Aufnahmetransportsysteme fehlen oder deren Aufnahmetransportsysteme beeinflußt sind.

17. Verfahren nach Anspruch 16, **dadurch gekennzeichnet, dass** vorzugsweise E.Coli Stämme ohne Transporter ProP und ProU, Bacillus-Arten ohne OpuD und/oder OpuE und Corynebacterium-Arten ohne EctP und/oder PröP als Empfängerorganismen herangezogen werden.

18. Verfahren nach Anspruch 16, **dadurch gekennzeichnet, dass** es sich um die Gene für Diaminobutyratacetylase (*ectA*), Diaminobutyrattransaminase (*ectB*) und Ectoinsynthase (*ectC*) aus *Marinoccus*- und/oder aus Bakterien der Familie Halomonadadeae, insbesondere aus *Halomonas*-Arten, oder aus anderen halophilen/halotoleranten Mikroorganismen handelt.

19. Verfahren nach Anspruch 18, **dadurch gekennzeichnet, dass** die Ectoingene mit Promotoren_{,} insbesondere lac, tac, T7, eingesetzt werden, die auch ohne osmotisches Signal induziert werden können oder Gene dauerhaft hoch exprimieren.

20. Verfahren nach Anspruch 16, **dadurch gekennzeichnet, dass** es sich bei dem Vektor um ein low-copy Plasmid handelt.

21. Verfahren nach Anspruch 16, **dadurch gekennzeichnet, dass** zur Vermeidung von Stoffwechselengpässen zusätzlich ein oder mehrere Gene auf die Produktionsstämme übertragen werden, die geeignet sind, den Stoffwechselengpass zu öffnen.

22. Verfahren nach Anspruch 21, **dadurch gekennzeichnet, dass** es sich bei den Genen zur Öffnung von Stoffwechselengpässen um Aspartokinasegene handelt, die aus halophilen Ectoin-synthetisierenden Organismen stammen oder mutiert oder dereguliert sind.

## Claims

1. Method of permanent production of ectoine and/or hydroxyectoine in organisms which have available information concerning the production of ectoine and/or hydroxyectoine and control mechanisms for control of production, **characterised in that** an existing control mechanism for limiting the biosynthesis in the cells is deleted, disabled and/or influenced in its function, wherein the control mechanism is the TRAP transported TeaABC.

2. Method as claimed in Claim 1, **characterised in that** the production strains are plant and/or animal cell cultures and yeasts, fungi or eucaryotic micro-organisms.

3. Method as claimed in Claim 1, **characterised in that** the production strains are procaryotes, in particular Archaea and bacteria.

4. Method as claimed in Claim 1, **characterised in that** the production strains are enterobacteria, in particular E. coli, bacillus species, Corynebacteria and micro-organisms from the family Halomonadaceae, preferably from the genus Halomonas, Marinococcus and related bacteria and Rhodothermus.

5. Method as claimed in Claim 1, **characterised in that** the synthesis is induced by means of raising or lowering the temperature or by means of lowering the water activity.

6. Method as claimed in Claim 1, **characterised in that** the synthesis is carried without induction at constantly high or temperature or at low temperature or at constantly low water activity.

7. Method as claimed in Claim 1, **characterised in that** the synthesis takes place by growing metabolically active cells.

8. Method as claimed in Claim 1, **characterised in that** the synthesis takes place in the form of a bioconversion with dormant or immobilised cells.

9. Method as claimed in Claim 1, **characterised in that** the valuable substances are separated from the production cells by means of permeable membranes, preferably by means of dialysis fermenters.

10. Method as claimed in Claim 1, **characterised in that** the uptake transport system is influenced or impaired by mutations by means of deletion, point mutation, insertions, influencing and/or inversions in the coding genes.

11. Method as claimed in Claim 1, **characterised in that** the uptake transport system is influenced or impaired by inhibition and/or blocking of the transport at the protein level by means of antimetabolites or chemicals.

12. Method as claimed in Claim 1, **characterised in that** the uptake transport system is influenced or impaired by manipulation of the energy supply of the producing cells.

13. Method as claimed in Claim 1, **characterised in that** it involves TeaABC from the family of Halomonadaceae, preferably from Halomonas species.

14. Method as claimed in Claim 1, **characterised in that** a regulator TeaD is assigned to the TeaABC, the TeaD being disabled as required.

15. Method as claimed in Claim 1, **characterised in that** the takeup transport systems are not active or are only partially active due to culture conditions and production conditions of the organisms.

16. Method as claimed in Claim 1, **characterised in that** the transfer of the nucleic acids, in particular genes or regulation sequences/promoters, takes place by means of vectors, and that it is autonomously replicated in the receiver organism or is permanently inserted into the genome, wherein the receiver organism involves micro-organisms which lack osmoregulated takeup transport systems or of which the uptake transport systems are influenced.

17. Method as claimed in Claim 16, **characterised in that** use is made of preferably E. coli strains without transporters ProP and ProOU, bacillus species without OpuD and/or OpuE and Corynebacterium species without EctP and/or ProP as receiver organisms.

18. Method as claimed in Claim 16, **characterised in that** it relates to the genes for diaminobutyrateacetylase (*ectA*), diaminobutyratetransaminase (*ectB*) and ectoine synthesis (*ectC*) from Mannococcus and/or from bacteria of the family Halomonadaceae, in particular from Halomonas species, or from other halophilic/halotolerant micro-organisms.

19. Method as claimed in Claim 18, **characterised in that** the ectoine genes are used with promoters, in particular lac, tac, T7, which can also be induced without an osmotic signal or permanently exhibit a high expression of genes.

20. Method as claimed in Claim 16, **characterised in that** the vector is a low-copy plasmid.

21. Method as claimed in Claim 16, **characterised in that** in order to avoid metabolic bottlenecks one gene or a plurality of genes which are suitable for opening the metabolic bottleneck are additionally transferred to the production strains.

22. Method as claimed in Claim 21, **characterised in that** the genes for opening of metabolic bottlenecks are aspartokinase genes which originate from halophilic ectoine-synthesising organisms or are mutated or deregulated.

## Revendications

1. Procédé de production permanente d'ectoïne et/ou d'hydroxyectoïne dans des organismes qui disposent d'informations génétiques sur la production d'ectoïne et/ou d'hydroxyectoïne et de mécanismes de régulation pour piloter la production, **caractérisé en ce qu'**un mécanisme de régulation présent destiné à limiter la biosynthèse dans les cellules est délété, supprimé et/ou affecté dans son fonctionnement, le mécanisme de régulation étant le transporteur de lacunes TeaABC.

2. Procédé selon la revendication 1, **caractérisé en ce que** les lignées de production sont des cultures cellulaires végétales et/ou animales et des levures, champignons ou micro-organismes eucaryotes.

3. Procédé selon la revendication 1, **caractérisé en ce que** les lignées de production sont des procaryotes, en particulier des archéobactéries et des bactéries.

4. Procédé selon la revendication 1, **caractérisé en ce que** les lignées de production sont des entérobactéries, en particulier E.coli, des variétés de bacilles, des corynebactéries et des micro-organismes de la famille des halomonadaceae, de préférence du genre Halomonas, des Marinococcus et des bactéries parentes, ainsi que des Rhodothermus.

5. Procédé selon la revendication 1, **caractérisé en ce que** la synthèse est induite par augmentation ou baisse de la température ou par diminution de l'activité de l'eau.

6. Procédé selon la revendication 1, **caractérisé en ce que** la synthèse est conduite sans induction à température constante élevée ou basse, ou s'effectue avec une activité de l'eau faible constante.

7. Procédé selon la revendication 1, **caractérisé en ce que** la synthèse s'effectue par des cellules croissantes métaboliquement actives.

8. Procédé selon la revendication 1, **caractérisé en ce que** la synthèse s'effectue sous la forme d'une bioconversion avec des cellules au repos resp. immobilisées.

9. Procédé selon la revendication 1, **caractérisé en ce que** les matériaux sont séparés des cellules de production au moyen d'une membrane perméable, de préférence au moyen d'un fermenteur à dialyse.

10. Procédé selon la revendication 1, **caractérisé en ce que** l'influence ou la modification sur le système de transport de réception s'effectue par des mutations au moyen d'une délétion, mutation ponctuelle, insertions, influence et/ou inversions dans les gènes codants.

11. Procédé selon la revendication 1, **caractérisé en ce que** l'influence ou la modification sur le système de transport de réception s'effectue par inhibition et/ou blocage du transport au niveau protéinique en utilisant des antimétabolites ou des produits chimiques.

12. Procédé selon la revendication 1, **caractérisé en ce que** l'influence ou la modification sur le système de transport de réception s'effectue par une manipulation de l'approvisionnement énergétique des cellules productrices.

13. Procédé selon la revendication 1, **caractérisé en ce qu'**il s'agit de TeaABC de la famille des halomonadaceae, de préférence de variétés d'Halomonas.

14. Procédé selon la revendication 1, **caractérisé en ce qu'**au TeaABC est associé un régulateur TeaD, celui-ci étant inhibé selon les besoins.

15. Procédé selon la revendication 1, **caractérisé en ce que**, du fait de conditions de croissance et de production des organismes, les systèmes de transport de récepteur ne sont pas actifs, ou seulement partiellement.

16. Procédé selon la revendication 1, **caractérisé en ce que** la transmission des acides nucléiques, en particulier de gènes resp. de séquences/promoteurs de régulation s'effectue au moyen de vecteurs, **en ce qu'**ils sont insérés dans le génome de manière durable ou par réplication autonome dans l'organisme récepteur, l'organisme récepteur étant des micro-organismes auxquels manquent des systèmes de transports de réception osmorégulés ou dont les systèmes de transport de réception sont influencés.

17. Procédé selon la revendication 16, **caractérisé en ce qu'**on utilise de préférence comme organismes récepteurs des souches E. Coli sans transporteur ProP et ProU, des variétés de bacilles sans OpuD et/ou OpuE et des variétés de corynebactéries sans EctP et/ou ProP.

18. Procédé selon la revendication 16, **caractérisé en ce qu'**il s'agit des gènes pour la diaminobutyratacétylase (ectA), diaminobutyrattransminase (ectB) et ectoïnesynthase (ectC) de Mannococcus et/ou de bactéries de la famille des halomonadaceae, en particulier de la variété Halomonas, ou d'autres microorganismes halophiles/halotolérants.

19. Procédé selon la revendication 18, **caractérisé en ce que** les gènes d'ectoïne sont utilisés avec des promoteurs, en particulier lac, tac, T7, qui peuvent être induits même sans signal osmotique ou qui expriment durablement des gènes.

20. Procédé selon la revendication 16, **caractérisé en ce que** le vecteur est un plasmide low-copy.

21. Procédé selon la revendication 16, **caractérisé en ce que**, pour éviter des goulots métaboliques, un ou plusieurs gènes sont transmis en plus sur les souches de production, qui conviennent pour ouvrir le goulot métabolique.

22. Procédé selon la revendication 21, **caractérisé en ce que** les gènes destinés à ouvrir les goulots métaboliques sont des gènes d'aspartocinase, qui proviennent d'organismes halophiles synthétisant l'ectoïne ou qui sont mutés ou dérégulés.
